# EUROPÄISCHE PATENTSCHRIFT

(11) **EP 2 398 437 B1**
(45) Veröffentlichungstag und Bekanntmachung des Hinweises auf die Patenterteilung: **08.04.2015**
(21) Anmeldenummer: 10708108.5
(22) Anmeldetag: 23.02.2010
(51) Int. Cl.: A61F 13/26

(54) **TAMPON-APPLIKATOR-ANORDNUNG**
TAMPON APPLICATOR ASSEMBLY
DISPOSITIF APPLICATEUR À TAMPON

(30) Priorität: 23.02.2009 AT 2942009
(43) Veröffentlichungstag der Anmeldung: 28.12.2011
(73) Patentinhaber: Ruggli Projects AG, 6332 Hagendorn (CH)
(72) Erfinder: TAMBURIN, Luca, 79801 Hohentengen (DE)
(74) Vertreter: Burger, Hannes
(86) Internationale Anmeldenummer: PCT/EP2010/001109
(87) Internationale Veröffentlichungsnummer: WO 2010/094507

(56) Entgegenhaltungen:
- EP-A1- 0 221 424
- EP-A2- 0 605 016
- US-A- 4 276 881
- US-A- 4 286 595
- US-A- 4 891 042
- US-A- 5 330 421

## Beschreibung

Die Erfindung bezieht sich auf eine Tampon-Applikator-Anordnung gemäß dem Oberbegriff des Anspruchs 1.

Darüber hinaus hat die Erfindung ein Verfahren gemäß Anspruch 7 zum Gegenstand.

Tamponapplikatoren zum Einführen von saugfähigen Tampons in Körperöffnungen sind seit langer Zeit bekannt. Bei einem Typ von Tamponapplikator ist der Tampon in einem Rohr angeordnet und es sind Ausstossmittel vorgesehen, um den Tampon aus dem Rohr auszustossen. Die Ausstossmittel können beispielsweise aus einem weiteren Rohr bestehen, das teleskopisch im erstgenannten Rohr verschiebbar ist. Ein Problem dieser bekannten Tamponapplikatoren besteht darin, dass sie wenn sie gebrauchsbereit ausgeliefert werden, relativ lang und damit unhandlich bei der Aufbewahrung und beim Transport sind. Zur Lösung dieses Problems wurden so genannte Kompaktapplikatoren entwickelt. Diese bestehen aus zwei teleskopischen Rohren, wobei der Tampon im Auslieferungszustand im Innenrohr und mit diesem zusammen im Aussenrohr angeordnet ist. Für den Gebrauch muss zuerst das Innenrohr zurückgezogen werden, um dann bei einer anschliessenden Vorschubbewegung hinten am Tampon anzugreifen und diesen aus dem Aussenrohr auszustossen. Damit der Tampon beim Zurückziehen des Innenrohrs nicht mitgenommen wird, sondern im Aussenrohr verbleibt, sind verschiedene Arten von Rückhaltemitteln bekannt.

Das Patent EP1704841B1 zeigt einen Kompaktapplikator, bei dem ein Tampon im Auslieferungszustand in einem Innenrohr aufgenommen ist, wobei er in Einführungsrichtung aus dem Innenrohr hinausragt. Das Innenrohr mit dem Tampon ist in einem Aussenrohr aufgenommen, welches am Einführungsende durch federnde Verschlussabschnitte kalottenförmig verschlossen ist. In der Nähe der Basis der Verschlussabschnitte sind am Aussenrohr nach innen gerichtete Vorsprünge angeordnet, welche am aus dem Innenrohr heraus ragenden Bereich des Tampons angreifen und den Tampon daran hindern, entgegen der Einführungsrichtung aus dem Aussenrohr herausgezogen zu werden, wenn das Innenrohr zurückgezogen wird.

Die Herstellung von Rückhaltemitteln, wie sie vorangehend beispielsweise beschrieben wurden, ist mit erheblichem Aufwand verbunden. Bei Applikatoren aus Kunststoff erhöhen solche Rückhaltemittel die Werkzeugkosten erheblich und bei Applikatoren aus Faserstoff, beispielsweise Karton ist die Herstellung von Rückhaltemitteln in der Gestalt von nach innen ragenden Vorsprüngen bei gleichzeitigem Vorhandensein von Verschlussabschnitten am Ende des Aussenrohrs praktisch unmöglich.

Im Dokument DE 69209085 T2 ist ein Kompaktapplikator beschrieben, welcher keine separaten Tamponrückhaltemittel aufweist, Das Zurückhalten des Tampons beim Zurückziehen des Innenrohrs wird durch den einführungsseitigen Rand des Aussenrohrs sowie eine Pilzgestalt der Tamponspitze gewährleistet. Ein vergrösserter Kopf des Tampons hindert den Tampon daran, in das äussere Rohr gezogen zu werden, wenn das innere Rohr zurückgezogen wird, um den Applikator für die Anwendung vorzubereiten. Dabei ragt aber das Einführungsende des Tampons schon im Auslieferungszustand aus dem äusseren Rohr hervor, was aus hygienischen Gesichtspunkten ungünstig ist.

Die EP 0 605 016 A2 betrifft einen Tampon-Applikator-Anordnung mit einem Aussenrohr und einem Innenrohr. Um ein Herausziehen des Tampons bei Zurückziehen des Innenrohrs aus dem Aussenrohr zu verhindern, weist das Aussenrohr einen nach innen ragenden Vorsprung auf

Die US 4,276,881 hat eine Tampon-Applikator-Anordnung zum Gegenstand, bei welcher ein Innenrohr teleskopartig in einem Aussenrohr verschiebbar ist. Um bei einem Zurückziehen des Innerohrs ein Herausrutschen des Tampons aus dem Aussenrohr zu verhindern weist, das Aussenrohr nach innen ragende Vorsprünge auf.

Das Dokument US 4,891,042 bezieht sich auf eine Tampon-Applikator-Anordnung mit einem Innen- und einem Aussenrohr, welche teleskopartig zueinander verschiebbar sind. Auch bei dieser Ausführungsform weist das Aussenrohr nach innen ragende Vorsprünge auf, welche bei Zurückziehen des Innerohres den Tampon in dem Aussenrohr zurückhalten.
Das Dokument US 4,286,595hat eine Tampon-Applikator-Anordnung zum Gegenstand, welche ein gegenüber einem Aussenrohr bewegbares Innenrohr aufweist. Das Aussenrohr ist mit nach innen ragenden Vorsprüngen 28 versehen, um bei Zurückziehen des Innenrohres den Tampon zurückzuhalten.

Die US 5 330 421 betrifft eine Tampon-Applikator-Anordnung mit einem Aussenrohr und einem Innenrohr. Das Aussenrohr wird aus einem Halterohr und einem mit diesem verklebten Rohr gebildet. Durch die Verklebung der Rohre wird anschließend an Verschlussabschnitte des Aussenrohres eine in das Innere des Aussenrohres abstehende Kante gebildet. Diese Kante, die einen von dem Außenrohr nach innen ragenden Vorsprung darstellt, dient dazu den Tampon bei einem Zurückziehen des teleskopartig in dem Aussenrohr bewegbaren Innerohres in dem Aussenrohr zu halten.

Ausgehend von diesem Stand der Technik ist es eine Aufgabe der Erfindung eine Tampon-Applikator-Anordnung vorzuschlagen, die einfach und kostengünstig herstellbar ist und bei welcher der Tampon vor Gebrauch vollständig im Applikator aufgenommen und dadurch gegen Berührung geschützt ist.

Diese Aufgabe wird erfindungsgemäss mit einer Vorrichtung gemäß Anspruch 1 bzw. einem Verfahren gemäß Anspruch 7 gelöst.

Diese erfindungsgemässe Lösung hat den Vorteil, dass am Ausserrohr keine nach innen ragende Vorsprünge angeordnet sein müssen, um den Tampon beim Zurückziehen des Innenrohrs im Aussenrohr zurückzuhalten. Beim Gebrauch wird der Tampon durch Vorschieben des Innenrohrs im Aussenrohr vorgeschoben, bis er die Verschlussabschnitte des Ausserrohrs nach aussen drängt und ein Stück aus dem Aussenrohr herausragt. Beim anschliessenden Zurückziehen des Innenrohrs wird der Tampon durch die Verschlussabschnitte des Aussenrohrs in seiner vorgeschobenen Position im Aussenrohr festgehalten. Durch den im Durchmesser erweiterten Kopf wird der vorgeschobene Tampon durch die Verschlussabschnitte des Aussenrohrs am Zurückziehen in das Aussenrohr gehindert, wenn das Innenrohr zurückgezogen wird.

Nach einer Ausführungsart der Erfindung sind die Verschlussabschnitte des Aussenrohrs durch vom Einführungsende des Aussenrohrs ausgehende Schlitze voneinander getrennt. Derart ausgebildete Verschlussabschnitte lassen sich sehr einfach herstellen.

Nach einer weiteren Ausführungsart ist im Bereich der vom Einführungsende entfernten Enden der Schlitze jeweils zwischen zwei Schlitzen eine in Umfangsrichtung des Aussenrohrs verlaufende Einkerbung angeordnet. Diese Einkerbungen wirken für den jeweils benachbarten Verschlussabschnitt als Scharnier, wenn dieser Verschlussabschnitt durch den durch ihn hindurch tretenden Tampon radial nach aussen gebogen wird.

Eine andere Ausführungsart sieht vor, dass im Bereich des vom Einführungsende entfernten Endes des Aussenrohrs ein Griffbereich ausgebildet ist, der aus an der äusseren Oberfläche des Aussenrohrs angeordneten Erhebungen und/oder Vertiefungen besteht. Dieser verhindert bei der Handhabung der Anordnung das Abrutschen mit den Fingern.

Gemäss einer weiteren Ausführungsart weist das Innenrohr an seinem Einführungsende mindestens zwei einstückig angeformte, nach innen weisende Verschlussabschnitte auf, die durch vom Einführungsende des Innenrohrs ausgehende Schlitze voneinander getrennt sind. Diese Verschlussabschnitte nehmen im Auslieferungszustand der Anordnung den Tampon zwischen sich auf und federn nach dem Zurückziehen des Innenrohrs bis hinter den im Aussenrohr festgehaltenen Tampon nach innen, so dass sie beim anschliessenden Vorschieben des Innenrohrs am hinteren Ende des Tampons anliegen.

Nach einer weiteren Ausführungsart ist am Innenrohr im Bereich der vom Einführungsende entfernten Enden der Schlitze jeweils zwischen zwei Schlitzen eine in Umfangsrichtung des Innenrohrs verlaufende Einkerbung angeordnet. Diese Einkerbungen wirken für den jeweils benachbarten Verschlussabschnitt als Scharnier, wenn dieser Verschlussabschnitt durch den durch ihn hindurch tretenden Tampon radial nach aussen gebogen wird.

Gemäss einer anderen Ausführungsart ist im Bereich des vom Einführungsende entfernten Endes des Innenrohrs ein Griffbereich ausgebildet, der aus an der äusseren Oberfläche des Innenrohrs angeordneten Erhebungen und/oder Vertiefungen besteht. Der Griffbereich verhindert insbesondere beim axialen Verschieben des Innenrohrs das Abrutschen mit den Fingern.

Nach einer bevorzugten Ausführungsart ist das Aussenrohr und/oder das Innenrohr aus einem faserhaltigen Material, insbesondere Karton hergestellt. Dieses Material ist kostengünstig in der Herstellung und Verarbeitung und lässt sich zudem umweltfreundlich entsorgen.

Die Erfindung wird im nachfolgenden anhand der in den Zeichnungen dargestellten Ausführungsbeispiele näher erläutert.

Es zeigen:
- Fig. 1: einen Tampon für eine erfindungsgemässe Tampon-Applikator-Anordnung;
- Fig. 2: ein Innenrohr zur Aufnahme des Tampons nach Fig. 1;
- Fig. 3: ein Aussenrohr zur Aufnahme des Innenrohrs nach Fig. 2;
- Fig. 4: eine Aussenansicht einer Tampon-Applikator-Anordnung aus den Komponenten gemäss den Figuren 1 bis 3 im Auslieferungszustand;
- Fig. 5: eine Schnittansicht der Anordnung nach Fig. 4;
- Fig. 6: eine perspektivische Ansicht der Anordnung im Auslieferungszustand;
- Fig. 7: eine perspektivische Ansicht der Anordnung nachdem das Innenrohr mit dem Tampon vorgeschoben wurde;
- Fig. 8: eine perspektivische Ansicht der Anordnung nachdem das Innenrohr ausgehend von der Position gemäss Fig. 7 zurückgezogen wurde;
- Fig. 9: eine perspektivische Ansicht der Komponenten nach dem vollständigen Ausstossen des Tampons.

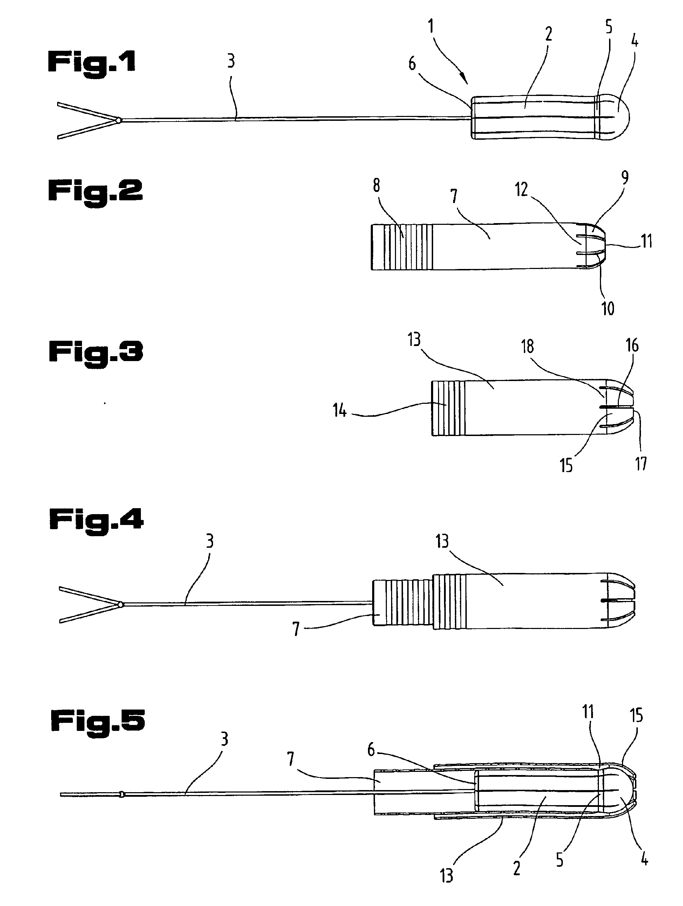

In der nachfolgenden Beschreibung wird bei allen Komponenten dasjenige Ende als Einführungsende bezeichnet, das dazu bestimmt ist, beim Gebrauch zuerst in eine Körperöffnung eingeführt zu werden. Das Einführungsende ist in allen Figuren 1 bis 9 rechts dargestellt. Entsprechend wird das dem Einführungsende gegenüberliegende Ende jeder Komponente als rückwärtiges Ende bezeichnet.

Fig. 1 zeigt ein Ausführungsbeispiel eines Tampons 1 für die Verwendung in einer erfindungsgemässen Anordnung. Beim Tampon kann es sich sowohl um einen relativ schwach komprimierten Tampon handeln, der nur mit Hilfe eines Applikators eingeführt werden kann oder aber auch um einen so genannten Digitaltampon, der so stark verdichtet ist, dass er eine Steifigkeit hat, die es erlaubt, ihn von Hand ohne Hilfe eines Applikators einzuführen. Der Tampon 1 besteht aus einem Tamponkörper 2 aus komprimiertem, saugfähigem Fasermaterial und hat eine Rückzugskordel 3, um den Tampon nach Gebrauch aus einer Körperhöhle heraus ziehen zu können. Der Tamponkörper 2 hat einen in etwa kreiszylindrischen Teil und am Einführungsende, in der Figur rechts, einen abgerundeten Kopf 4, dessen Aussendurchmesser etwas grösser ist als der kreiszylindrische Teil des Tamponkörpers 2. Zwischen dem kreiszylindrischen Teil und dem Kopf 4 ist eine ringförmige Anlagefläche 5 gebildet, auf deren Funktion später noch eingegangen wird. Dem Kopf 4 gegenüberliegend hat der Tamponkörper 2 eine rückseitige Stirnfläche 6.

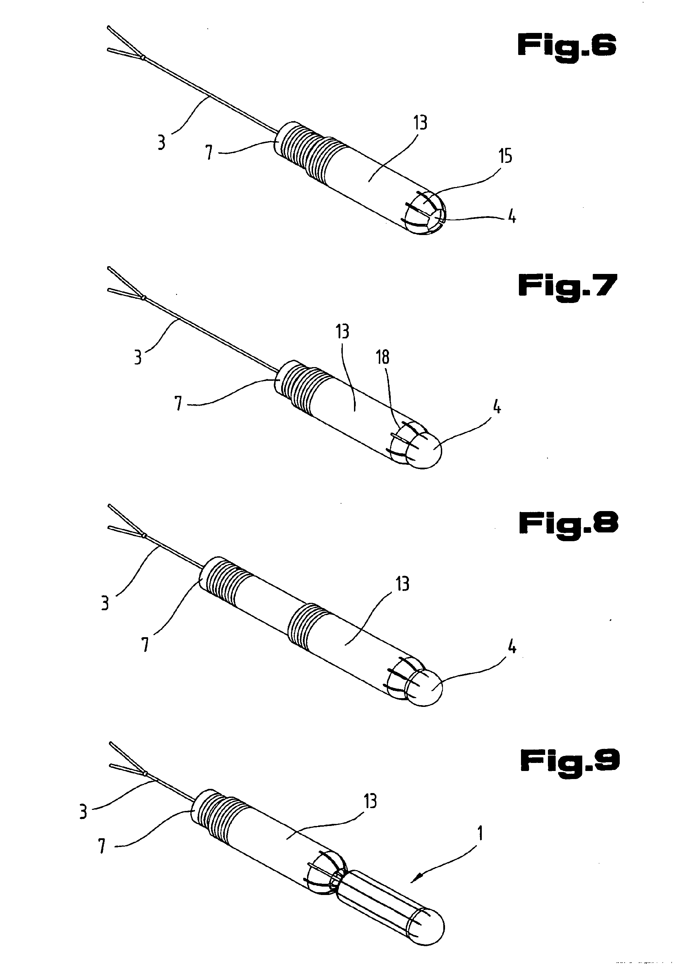

Fig. 2 zeigt ein Beispiel eines Innenrohrs 7 für eine erfindungsgemässe Anordnung. Das Innenrohr 7 hat vorzugsweise nahe beim Rückzugsende auf seiner äusseren Oberfläche einen Griffbereich 8, der die Aufgabe hat, beim axialen Verschieben des Innenrohrs 7 im nachstehend beschriebenen Aussenrohr 13 das Abrutschen mit den Fingern zu verhindern. Der Griffbereich 8 besteht aus an der äusseren Oberfläche des Innenrohres 7 angeordneten Erhebungen und/oder Vertiefungen, die beispielsweise durch Prägen gebildet sein können. Im dargestellten Beispiel besteht der Griffbereich 8 aus abwechselnd angeordneten umlaufenden Rillen und Rippen. Am Einführungsende ist das Innenrohr 7 durch eine Anzahl von Verschlussabschnitten 9 mindestens teilweise verschlossen. Die Verschlussabschnitte 9 sind durch Schlitze 10 aus dem Material des Innenrohrs gebildet und so nach innen gebogen, dass ihre Oberflächen in etwa die Form eines Kugelabschnitts haben. Es sind mindestens zwei Schlitze 10 vorhanden, im dargestellten Beispiel sind es acht Stück. Die freien Enden der Verschlussabschnitte 9 sind vorzugsweise nicht ganz spitz, sondern haben eine rechtwinklig zur Längsachse des Innenrohrs 7 verlaufende Endkante 11. Dadurch verbleibt am Einführungsende eine kreisrunde Öffnung (siehe Fig. 6) und das Einführungsende hat folglich eher die Form einer Kugelzone. Damit sich die Verschlussabschnitte 9 bei Bedarf leichter nach aussen biegen lassen, kann in deren Basisbereich im Innenrohr 7 eine umlaufende Einkerbung 12 vorgesehen sein, die wie ein Scharnier wirkt.

In Fig. 3 ist ein Beispiel eines Aussenrohrs 13 für eine erfindungsgemässe Anordnung dargestellt. Das Aussenrohr 13 ist prinzipiell gleich aufgebaut wie das Innenrohr 7, ist jedoch etwas kürzer und hat einen grösseren Durchmesser, so dass das Aussenrohr 13 das Innenrohr 7 teleskopisch in sich aufnimmt. Auch das Aussenrohr 13 hat einen Griffbereich 14 und am Einführungsende angeordnete Verschlussabschnitte 15, die durch Schlitze 16 aus dem Material des Aussenrohrs 13 gebildet sind. Es sind mindestens zwei Schlitze 16 vorhanden, im dargestellten Beispiel sind es acht Stück. Die Schlitze 16 gehen vom Einführungsende des Aussenrohrs aus und verlaufen im dargestellten Beispiel parallel zur Längsachse des Aussenrohrs. Sie können aber auch in einem Winkel zur Längsachse verlaufen, also die Form einer Schraubenlinie aufweisen. Dies gilt analog auch für die Schlitze 10 im Innenrohr 7. Wie beim Innenrohr 7 können auch beim Aussenrohr 13 die Verschlussabschnitte 15 eine Endkante 17 haben und durch eine Einkerbung 18 leichter beweglich sein.

Das Innenrohr 8 und das Aussenrohr 13 bestehen vorzugsweise aus Karton und sind beispielsweise durch schraubenförmiges Wickeln und Verkleben aus streifenförmigem Material gebildet.

Die Figuren 4 und 5 zeigen die Tamponanordnung in zusammengebautem Zustand, Fig. 4 in einer Aussenansicht und Fig. 5 in einer Schnittansicht. Das Zusammenfügen der Komponenten wird ausgeführt, indem zuerst der Tampon 1 vom rückwärtigen Ende her in das Innenrohr 7 eingeführt wird, und zwar so weit, bis der Kopf 4 des Tampons die Verschlussabschnitte 9 am Einführungsende des Innenrohrs 7 nach aussen drängt und am Einführungsende aus dem Innenrohr herausragt. Wie in Fig. 5 zu erkennen ist, liegen die Endkanten 11 der Verschlussabschnitte 9 auf Grund ihrer Rückfederungseigenschaften auf der Aussenfläche des Tamponkörpers 2 an. Anschliessend wird das Innenrohr 7 mit dem darin aufgenommenen Tampon 1 vom rückwärtigen Ende des Aussenrohrs 13 in dieses eingeführt, und zwar höchstens so weit, bis der Kopf 4 des Tampons an der Innenfläche der Verschlussabschnitte 15 anliegt. In dieser gegenseitigen Lage der Komponenten, wie sie in den Figuren 4 und 5 dargestellt ist, wird die Anordnung verpackt und ausgeliefert. Einer der Vorteile der Erfindung besteht darin, dass der Tampon im Auslieferungszustand der Anordnung vollständig vom Applikator umschlossen ist.

In den Figuren 6 bis 9 sind die Vorgänge beim Gebrauch der erfindungsgemässen Anordnung dargestellt. Fig. 6 zeigt die Anordnung im Auslieferungszustand, der vorangehend unter Bezugnahme auf die Figuren 4 und 5 beschrieben wurde. Wie in Fig. 6 gut zu erkennen ist, verbleibt an der Spitze des Aussenrohrs 13 eine kreisrunde Öffnung, wenn die Verschlussabschnitte 15 nicht spitz zulaufen, sondern eine Endkante 17 besitzen. Bevor bei der Benutzung der Anordnung das Innenrohr 7 aus dem Aussenrohr 13 zurückgezogen wird, muss das Innenrohr 7 zuerst so weit in Einführungsrichtung vorgeschoben werden, bis der Kopf 4 des Tampons 1 aus dem Aussenrohr 13 heraus ragt, wie dies in Fig. 7 dargestellt ist. Dieser Schritt kann vor oder nach Einführung der Vorrichtung in eine Körperöffnung erfolgen. Letzteres ist aus hygienischen Gesichtspunkten günstiger und insbesondere auch dann besonders empfehlenswert, wenn der Tampon einen Wirkstoff enthält, der erst mit dem Körpergewebe in Kontakt kommen sollte, wenn sich der Tampon nahezu an seinem Bestimmungsort im Körper befindet.

Ausgehend von der in Fig. 7 dargestellten Position wird nun das Innenrohr 7 zurückgezogen. Dabei besteht die Tendenz, dass der im Innenrohr aufgenommene Tampon 1 in Folge der zwischen der Aussenfläche des Tampons und der Innenfläche des Aussenrohrs 7 auftretenden Reibung mit nach hinten gezogen wird. Dies wird jedoch durch die Verschlussabschnitte 15 des Aussenrohrs 13 verhindert, die an der Mantelfläche des Tamponkörpers 2 und an der Anlagefläche 5 angreifen und den Tampon in seiner Position bezüglich des Aussenrohrs 13 festhalten. Das Innenrohr 7 wird in der Folge so weit nach hinten bewegt, bis sich sein Einführungsende hinter der rückseitigen Stirnfläche 6 des Tamponkörpers befindet. In diesem Moment werden die Verschlussabschnitte 9 des Innenrohrs auf Grund ihrer elastischen Vorspannung nach innen zurückfedern. Dieser Vorgang ist deutlich spürbar, weil der dem Zurückziehen des Innenrohrs 7 entgegen wirkende, durch die Reibung zwischen dem Innenrohr und dem Tampon verursachte Widerstand plötzlich nachlässt. Dies ist das Zeichen, das Zurückziehen des Innenrohrs zu beenden. Es ist auch in der nun erreichten, in Fig. 8 dargestellten Position noch möglich, die Anordnung in eine Körperöffnung einzuführen, falls dies nicht schon vorher geschehen ist.

Ausgehend von der in Fig. 8 dargestellten Position wird das Innenrohr 7 in Einführungsrichtung bewegt, wobei die einwärts gerichteten Verschlussabschnitte 9 des Innenrohrs 7 an der rückseitigen Stirnfläche 6 des Tamponkörpers 2 zur Anlage gelangen und folglich der Tampon 1 aus dem Aussenrohr 13 ausgestossen wird, wie dies in Fig. 9 dargestellt ist.

Der Ordnung halber sei abschliessend darauf hingewiesen, dass zum besseren Verständnis des Aufbaus der Anordnung diese beziehungsweise deren Komponenten teilweise unmassstäblich und/oder vergrössert und/oder verkleinert dargestellt wurden.

## Patentansprüche

1. Tampon-Applikator-Anordnung mit einem Einführungsende und einem rückwärtigen Ende, enthaltend einen Tampon (1) aus saugfähigem Material, mit einem Tamponkörper (2), der einen kreiszylindrischen Teil und am Einführungsende einen abgerundeten Kopf (4) mit einem etwas größeren Außendurchmesser als der kreiszylindrische Teil aufweist, wobei zwischen dem kreiszylindrischen Teil und dem Kopf (4) eine ringförmige Anlagefläche (5) gebildet ist, und einen Applikator bestehend aus einem Aussenrohr (13) und einem darin teleskopisch verschiebbaren Innenrohr (7), wobei das Aussenrohr (13) an seinem Einführungsende mindestens zwei einstückig angeformte, nach innen weisende Verschlussabschnitte (15) aufweist, wobei die Verschlussabschnitte (15) des Aussenrohrs (13) durch vom Einführungsende des Aussenrohrs ausgehende Schlitze (16) voneinander getrennt sind und wobei der Tampon (1) mit seinem Einführungsende aus dem Einführungsende des Innenrohrs (7) ragt und in einem Auslieferungszustand vollständig innerhalb des Aussenrohrs (13) aufgenommen ist, wobei das Innenrohr (7) an seinem Einführungsende mindestens zwei einstückig angeformte, nach innen weisende Verschlussabschnitte (9) aufweist, die durch vom Einführungsende des Innenrohrs ausgehende Schlitze (10) voneinander getrennt sind, **dadurch gekennzeichnet, dass** das Innenrohr (7) bevor es aus dem Außenrohr (13) zurückgezogen wird eine vorgeschobene Position aufweist, in der der Kopf (4) des Tampons (1) die Verschlussabschnitte (15) des Aussenrohres (13) überragt, wobei die Verschlussabschnitte (15) des Aussenrohres (13) an der Mantelfläche des Tamponkörpers (2) und an der Anlagefläche (5) angreifen und den Tampon (1) in seiner Position bezüglich des Aussenrohres (13) festhalten und eine zurückgezogene Position des Innenrohrs ausgebildet ist, in der die Verschlussabschnitte (9) des Innenrohres (7) in einer nach innen zurückgefederten Position hinter einer hinteren Stirnseite (6) des Tamponkörpers (2) zum Ausstoßen eines Tampons (1) aus dem Außenrohr (13) angeordnet sind und die innere Oberfläche des Aussenrohrs (13) ausgehend von den Verschlussabschnitten (15) glattwandig ohne nach innen ragende Vorsprünge ausgebildet ist.

2. Tampon-Applikator-Anordnung nach Anspruch 1, **dadurch gekennzeichnet, dass** im Bereich der vom Einführungsende entfernten Enden der Schlitze (16) jeweils zwischen zwei Schlitzen (16) eine in Umfangsrichtung des Aussenrohrs (13) verlaufende Einkerbung (18) angeordnet ist.

3. Tampon-Applikator-Anordnung nach einem der vorangehenden Ansprüche, **dadurch gekennzeichnet, dass** im Bereich des vom Einführungsende entfernten Endes des Aussenrohrs (13) ein Griffbereich (14) ausgebildet ist, der aus an der äusseren Oberfläche des Aussenrohrs (13) angeordneten Erhebungen und/oder Vertiefungen besteht.

4. Tampon-Applikator-Anordnung nach Anspruch 3, **dadurch gekennzeichnet, dass** am Innenrohr (7) im Bereich der vom Einführungsende entfernten Enden der Schlitze (10) jeweils zwischen zwei Schlitzen (10) eine in Umfangsrichtung des Innenrohrs (7) verlaufende Einkerbung (12) angeordnet ist.

5. Tampon-Applikator-Anordnung nach einem der vorangehenden Ansprüche, **dadurch gekennzeichnet, dass** im Bereich des vom Einführungsende entfernten Endes des Innenrohrs (7) ein Griffbereich (8) ausgebildet ist, der aus an der äusseren Oberfläche des Innenrohrs (7) angeordneten Erhebungen und/oder Vertiefungen besteht.

6. Tampon-Applikator-Anordnung nach einem der vorangehenden Ansprüche, **dadurch gekennzeichnet, dass** das Aussenrohr (13) und/oder das Innenrohr (7) aus einem faserhaltigen Material, insbesondere Karton hergestellt ist.

7. Verfahren zum Positionieren eines Tampons in einer Tampon-Applikator-Anordnung gemäß einem der Ansprüche 1 bis 6, gekennzeichnet durch folgende Schritte:
i) Einführen des Tampons (1) vom rückwärtigen Ende her in das Innenrohr (7) so weit, bis ein Kopf (4) des Tampons (1) die Verschlussabschnitte (9) am Einführungsende des Innenrohrs (7) nach aussen drängt und am Einführungsende aus dem Innenrohr (7) herausragt;
ii) Einführen des Innenrohrs (7) mit dem darin aufgenommenen Tampon (1) vom rückwärtigen Ende des Aussenrohrs (13);
iii) Vorschieben des Innenrohrs (7) in dem Ausssenrohr (13) soweit, bis der Kopf (4) des Tampons (1) aus dem Aussenrohr (13) hervorragt, bevor das Innenrohr (7) aus dem Aussenrohr (13) zum Ausstoßen des Tampons (1) zurückgezogen wird;
iv) Angreifen der Verschlussabschnitte (15) des Aussenrohrs (13) an einer Mantelfläche des Tamponkörpers (2) und an einer Anlagefläche (5) des Tampons (1) und Festhalten des Tampons (1) mittels der Verschlussabschnitte (15) in seiner Position bezüglich des Aussenrohrs (13).

## Claims

1. Tampon applicator assembly with an insertion end and a rear end containing a tampon (1) made from absorbent material, with a tampon body (2) having a circular cylindrical part and, at the insertion end, a rounded head (4) the external diameter of which is slightly bigger than the circular cylindrical part, with an annular abutment surface (5) disposed between the circular cylindrical part and the head (4), and an applicator comprising an outer tube (13) and an inner tube (7) able to slide telescopically in it, and the outer tube (13) has at its insertion end at least two integrally formed, inwardly directed closure portions (15), and the closure portions (15) of the outer tube (13) are separated from one another by slots (16) extending from the insertion end of the outer tube, and the tampon (1) is adapted to protrude out from the insertion end of the inner tube (7) by means of its insertion end and is, in its state as sold, accommodated completely inside the outer tube (13), and the inner tube (7) has at its insertion end at least two integrally formed, inwardly directed closure portions (9) which are separated from one another by slots (10) extending from the insertion end of the inner tube, **characterized in that** the inner tube (7) before being pulled out of the outer tube (13) is in a pushed-forward position in which position the head (4) of the tampon (1) protrudes beyond the closure portions (15) of the outer tube (13), and the closure portions (15) of the outer tube (13) grip on the external surface of the tampon body (2) and on the abutment surface (5) and hold the tampon (1) firmly in its position relative to the outer tube (13) and a pulled-back position of the inner tube is formed in which position the closure portions (9) of the inner tube (7) are arranged in a position sprung back inwardly behind an rear end face (6) of the tampon body (2) for ejecting the tampon (1) from the outer tube (13) and the outer tube (13) has a smooth, internal wall extending from the closure portions (15) without any inwardly extending projections.

2. Tampon applicator assembly according to claim 1, **characterized in that** an indentation (18) extending in the circumferential direction of the outer tube (13) is provided in the region of the ends of the slots (16) remote from the insertion end respectively between two slots (16).

3. Tampon applicator assembly according to one of the preceding claims, **characterized in that** a gripping region (14) is provided in the region of the end of the outer tube (13) remote from the insertion end, comprising raised areas and/or recesses disposed in the external surface of the outer tube (13).

4. Tampon applicator assembly according to claim 3, **characterized in that** an indentation (12) extending in the circumferential direction of the inner tube (7) is provided on the inner tube (7) in the region of the ends of the slots (10) remote from the insertion end respectively between two slots (10).

5. Tampon applicator assembly according to one of the preceding claims, **characterized in that** a gripping region (8) is provided in the region of the end of the inner tube (7) remote from the insertion end, comprising raised areas and/or recesses disposed in the external surface of the inner tube (7).

6. Tampon applicator assembly according to one of the preceding claims, **characterized in that** the outer tube (13) and/or the inner tube (7) is made from a fiber-containing material, in particular cardboard.

7. Method for positioning a tampon in a tampon applicator assembly according to one of claims 1 to 6, **characterized by** the following steps:
i) inserting the tampon (1) in the inner tube (7) from the rear end far enough for a head (4) of the tampon (1) to force the closure portions (9) at the insertion end of the inner tube (7) outwards and protrude out of the inner tube (7) at the insertion end;
ii) inserting the inner tube (7) with the tampon (1) accommodated in it from the rear end of the outer tube (13);
iii) pushing the inner tube (7) in the outer tube (13) far enough for the head (4) of the tampon (1) to protrude out of the outer tube (13) before the inner tube (7) is pulled back out of the outer tube (13) for ejecting the tampon (1);
iv) the closure portions (15) of the outer tube (15) gripping on an external surface of the tampon body (2) and an abutment surface (5) of the tampon (1) and holding the tampon (1) firmly in its position relative to the outer tube (13) by means of the closure portions (15).

## Revendications

1. Dispositif applicateur de tampon avec une extrémité d'introduction et une extrémité carrière, comprenant un tampon (1) en matériau absorbant, avec un corps de tampon (2), qui comprend une partie cylindrique et, à l'extrémité d'introduction, une tête arrondie (4) avec un diamètre extérieur un peu supérieur à celui de la partie cylindrique, une surface d'appui annulaire (5) étant formée entre la partie cylindrique et la tête (4) et un applicateur constitué d'un tube externe (13) et d'un tube interne (7) coulissant télescopique, le tube externe (13) comprenant, à son extrémité d'introduction, au moins deux portions de fermeture (15) moulées d'une seule pièce et orientées vers l'intérieur, ces portions de fermeture (15) du tube externe (13) étant séparées l'une de l'autre par des fentes (16) partant de l'extrémité d'introduction du tube externe et le tampon (1) dépassant, avec son extrémité d'introduction, de l'extrémité d'introduction du tube interne (7) et étant, dans un état de livraison, entièrement logé à l'intérieur du tube externe (13), le tube interne (7) comprenant, à son extrémité d'introduction, au moins deux portions de fermeture (9) moulées d'une seule pièce et orientées vers l'intérieur, qui sont séparées l'une de l'autre par des fentes (10) parant de l'extrémité d'introduction du tube interne, **caractérisé en ce que** le tube interne (7), avant d'être retiré du tube externe (13), présente une position poussée en avant, dans laquelle la tête (4) du tampon (1) dépasse des portions de fermeture (15) du tube externe (13), les portions de fermeture (15) du tube externe (13) s'accrochant à la surface externe du corps de tampon (2) et à la surface d'appui (5) et maintenant le tampon (1) dans sa position par rapport au tube externe (13) et une position rétractée du tube interne étant conçue, dans laquelle les portions de fermeture (9) du tube interne (7) sont disposées dans une position amortie en retour vers l'intérieur derrière un côté frontal arrière (6) du corps de tampon (2) pour l'éjection d'un tampon (1) hors du tube externe (13) et la surface interne du tube externe (13) est conçu, à partir des portions de fermeture, avec une paroi lisse sans saillies dépassant vers l'intérieur.

2. Dispositif applicateur de tampon selon la revendication 1, **caractérisé en ce que**, au niveau des extrémités des fentes (16) éloignées de l'extrémité d'introduction, entre deux fentes (16), se trouve une entaille (18) s'étendant sur la circonférence du tube externe (13).

3. Dispositif applicateur de tampon selon l'une des revendications précédentes, **caractérisé en ce que**, au niveau des extrémités du tube externe (13) éloignées de l'extrémité d'introduction, une zone de préhension (14) est réalisée, qui est constituée de bossages et/ou de creux disposés sur la surface extérieure du tube externe (13).

4. Dispositif applicateur de tampon selon la revendication 3, **caractérisé en ce que**, sur le tube interne (7), au niveau des extrémités des fentes (10) éloignées de l'extrémité d'introduction, entre deux fentes (10), se trouve une entaille (12) s'étendant sur la circonférence du tube interne (7).

5. Dispositif applicateur de tampon selon l'une des revendications précédentes, **caractérisé en ce que**, au niveau des extrémités du tube interne (7) éloignées de l'extrémité d'introduction, une zone de préhension (8) est réalisée, qui est constituée de bossages et/ou de creux disposés sur la surface extérieure du tube externe (7).

6. Dispositif applicateur de tampon selon l'une des revendications précédentes, **caractérisé en ce que** le tube externe (13) et/ou le tube interne (7) est constitué d'un matériau fibreux, plus particulièrement du carton.

7. Procédé de positionnement d'un tampon dans un dispositif applicateur de tampon selon l'une des revendications 1 à 6, **caractérisé par** les étapes suivantes:
i) introduction du tampon (1) de l'extrémité arrière vers le tube interne (7) jusqu'à ce qu'une tête (4) du tampon (1) pousse les portions de fermeture (9) à l'extrémité d'introduction du tube interne (7) vers l'extérieur et dépasse, au niveau de l'extrémité d'introduction, hors du tube interne (7);
ii) introduction du tube interne (7), avec le tampon (1) contenu à l'intérieur, à partir de l'extrémité arrière du tube externe (13);
iii) poussée du tube interne (7) dans le tube externe (13) jusqu'à ce que la tête (4) du tampon (1) dépasse du tube externe (13), avant que le tube interne (7) soit retiré du tube externe (13) pour l'éjection du tampon (1);
iv) préhension des portions de fermeture (15) du tube externe (13) au niveau d'une surface externe du corps de tampon (2) et au niveau d'une surface d'appui (5) du tampon (1) et maintien du tampon (1) à l'aide des portions de fermeture (15) dans sa position par rapport au tube externe (13).
